# EUROPEAN PATENT APPLICATION

(11) **EP 4 819 037 A1**
(43) Date of publication of application: **07.10.2026**
(21) Application number: 25167362.0
(22) Date of filing: 31.03.2025
(51) Int. Cl.: G06T 7/00, G06T 7/11

(54) **A MULTI-MODAL APPROACH FOR GRADING AN ARTERIOVENOUS MALFORMATION**

(71) Applicant: Siemens Healthineers AG, 91301 Forchheim (DE)
(72) Inventor: ABDISHEKTAEI, Mohammad, Charlottesville, VA 22903 (US); KOLLERATHU, Varghese, 560066 Whitefield, Bengalurur, Karnataka (IN); RAMESH, Rahul, 560064 Avalahalli, Bengaluru (IN); TEJANI, Abhinandan, 560068 Kudlu, Bangalore (IN); VINAY BHOMBORE, Vineet, 560078 Bengaluru, Karnataka (IN); WOLF, Matthias, Coatesville, PA 19320 (US); YEREBAKAN, Halid, Carmel, IN 46033 (US)
(74) Representative: HKW Intellectual Property PartG mbB

(57) **Abstract**

The invention relates to a computer-implemented method for characterizing a lesion in a blood vessel of a brain depicted in a radiography image comprising identifying a void, in the radiography image, wherein the void is representative for a presence of the lesion, determining a distance of the identified void to a region of interest in the radiography image, determining a size of a nidus in the radiography image, determining a presence of a deep venous drainage in the radiography image and determining a grading of the lesion based on the determined distance of the void to the region of interest, based on the size of the nidus and/or based on the determined presence of the deep venous drainage.

## Description

The present invention relates to a computer-implemented method, a computer-implemented apparatus, a system and a computer-program product for characterizing a lesion in a blood vessel of a brain depicted in a radiography image.

Medical imaging has provided a powerful tool for diagnosing abnormalities in a body of a patient for many years and it has developed to represent a standard procedure in state-of-the-art medical diagnostics. Medical imaging made it possible to support a non-invasive diagnosis based on images that were captured of certain parts of a body of a patient. Said images were, e.g., captured by means of X-ray imaging, computed tomography (CT) imaging, positron emission tomography (PET) imaging, ultrasonic imaging, etc.

The imaging quality of these imaging methods has drastically improved over the last decades supporting the accurate identification of ever decreasing abnormalities. The imaging quality has further improved such that it allows precise capturing of, e.g., X-rays which have penetrated the body of the patient with an improved contrast ratio (as compared to respective devices used decades ago) and suppressed blurring effects. These improvements in the imaging quality have advantageously contributed to a general improvement of medical imaging and has led to an increase in the constant demand for medical imaging even further.

However, even nowadays, the acquired radiography images (such as, e.g., magnetic resonance imaging (MRI)) are, to a wide extent, still analyzed manually by an experienced physician. This is time and resource intensive and leads to high costs of medical imaging which, in turn, need to be compensated by already weakened healthcare systems and are accompanied by prolonged waiting times for the patients requiring a medical imaging-based diagnosis.

The detection of small anomalies in the body of a patient plays a dominant role when small blood vessels need to be investigated. These small-scale structures can oftentimes not be reliably identified based on currently existing (mostly manual) methods of reviewing medical images. However, even though some blood vessels may be small, they may nevertheless be subject to severe and threatening diseases or may even be their origin.

This may be of relevance when lesions of the blood vessels are formed, leading to arteriovenous malformations (AVMs) formed by a group of blood vessels. In such malformations, arteries and veins may unusually be tangled and may form direct connections, bypassing normal blood vessels.

AVM is a neurovascular disorder wherein there is an absence of capillaries in blood vessels. This may lead to a low resistance pathway for the blood stream. AVMs are the second largest cause of cerebral haemorrhage. Children tend to be more susceptible to cerebral haemorrhage and the associated mortality rate is 10-15%.

The tangled formations may also be referred to as a *nidus* of an AVM. The identification of such a nidus may be seen as crucial to prevent a deterioration of the health state of a patient which may arise from the presence of the nidus.

What is more, even if a nidus is detected, a decision to be made by a medical practitioner is whether to treat the underlying AVM or not. However, up to today, a demarcation of arteries and veins in respective radiography images is mainly done manually. This may require reviewing a plurality of angiography images, identifying arteries and veins manually and deciding based thereon, whether a nidus (e.g., of harmful size) is present or not.

Even if it is determined that a lesion (e.g., based on a nidus) is present in a radiography image, it still requires an additional manual step to decide whether the lesion is to be treated (e.g., by surgery) or not.

In a clinical setting, when it is required to decide whether a lesion is to be treated, medical practitioners may make use of guidelines and pre-defined grading systems such as a Spetzler-Martin grading system for quantifying a lesion. For example, patients with lower grades (I and II) according to the Spetzler-Martin grading system may represent suitable surgical candidates, as they may have a lower risk of surgery-related neurological deficits. In contrast, patients with higher grades (IV and V) according to the Spetzler-Martin grading system may be at a high risk of surgical complications and may usually be managed conservatively or with non-surgical methods like stereotactic radiosurgery or embolization.

Therefore, there is still a need to improve the analysis of radiography images, in particular, when it comes to the analysis of small-scale structures, such as, e.g., blood vessels, which is currently not yet fulfilled in a satisfying manner based on the presently known and applied procedures.

Therefore, it is an object of the present invention to further improve the characterization of lesions.

According to a first aspect, a computer-implemented method for characterizing a lesion in a blood vessel of a brain depicted in a radiography image is suggested. The computer-implemented method may comprise identifying a void in the radiography image, wherein the void is representative for a presence of the lesion, determining a distance of the identified void to a region of interest in the radiography image and determining a size of a nidus in the radiography image. Moreover, the computer-implemented method may comprise determining a presence of a deep venous drainage in the radiography image and determining a grading of the lesion based on the determined distance of the void to the region of interest, based on the size of the nidus and/or based on the determined presence of the deep venous drainage.

In some examples, the void may be associated with (e.g., may represent) a location of the lesion.

By determining the grading of the lesion based on the determined distance of the void to the region of interest, based on the size of the nidus and/or based on the determined presence of the deep venous drainage, a multi-modal computer-implemented (and thus automated) method may be provided to efficiently characterize a lesion in a blood vessel of a brain of a patient. This may generally support a faster characterization of the lesion and may support a more accurate diagnosis of the respective lesion as well as a decision whether to treat (e.g., by surgery) or not. Moreover, a multi-modal characterization of the lesion may be provided as more than one parameter may be considered when determining the grading.

In an embodiment, the identifying of the void may comprise identifying the void based on a trained artificial intelligence model, preferably by a trained classification model, more preferably by a Residual Network, ResNet.

In case no void is detected in the radiography image, it may be concluded that no lesion is present in the radiography image. In such a case, the computer-implemented method may be terminated. In some examples, a standard value (e.g., a logical 0 or 1) may be output by the computer-implemented method, indicating that no void was detected in the radiography image.

A classification model may be referred to as a type of artificial intelligence model designed to categorize or group data into predefined classes or categories based on specific features or characteristics. A respective classification model may learn patterns and relationships from labeled training data, where each data point may be associated with a known class. Once trained, classification models may predict the class of new, unseen data points by analyzing their features and applying the learned patterns.

Classification models may be configured to handle different types of classification tasks, including binary classification (where data is sorted into two classes) and multi-class classification (involving more than two classes). Prior to applying a classification model for the actual classification task, preceding training of the model on a labeled dataset may occur, evaluating its performance on test data, and then applying it to real-world scenarios to classify new, unseen instances.

A ResNet may be referred to as a deep learning artificial intelligence model designed to address the challenges of training (deep) artificial intelligence models, such as neural networks. It may be based on the concept of residual learning, where each layer of the deep neural network may learn residual functions with reference to the layer inputs, rather than learning unreferenced functions. This approach may allow for the creation of much deeper networks without suffering from a vanishing gradient problem. This may be facilitated by incorporating skip connections, also known as shortcut connections, which may enable the network to bypass one or more layers. These connections may facilitate the flow of information across the neural network, making it easier to train and optimize very deep models.

This may generally support an automated, accurate and thus efficient identifying of a void in the radiography image under investigation.

In another embodiment, the region of interest may be an eloquent area of the brain.

An eloquent area of the brain may be referred to an area that is responsible for functions of the patient like movement, sensation and/or speech.

By determining a distance from the void to the region of interest, a risk indicator may be derived which may indicate a risk for treating the void by surgery.

In another embodiment, the determining of the distance may comprise mapping an atlas of the brain to the radiography image, providing the mapped radiography image to a trained artificial intelligence model which is trained for localizing a lesion in a radiography image and localizing the lesion in the radiography image, based on the respectively trained artificial intelligence model, wherein the localizing preferably comprises determining coordinates of the lesion.

A lesion may be referred to as an anomaly in a tissue depicted in the radiography image.

The mapped radiography image may be provided with an atlas-based annotation of anatomical regions/nuclei of the brain of the patient.

In some examples, the atlas may represent anatomical regions of the (human) brain. In some examples, the atlas may be a Morel atlas.

The coordinates may indicate the location of the lesion in a cartesian coordinate system, e.g., in a two-dimensional coordinate system (X-Y-coordinate system) or in a three-dimensional coordinate system (X-Y-Z-coordinate system).

In some examples, the artificial intelligence model, trained for localizing the lesion, may be a convolutional neural network (CNN), e.g., comprised by a U-net architecture.

The coordinates may indicate a center of the lesion. The center may be a point comprised by the lesion that has a same distance to each point of a demarcation line surrounding the lesion.

In another embodiment, the mapping of the atlas may comprise a rigid mapping.

A rigid mapping may be referred to as a type of transformation that preserves the shape and size of an object, only allowing for changes in position and orientation. The rigid mapping may be used to align or transform information from the atlas while maintaining its fundamental structure. Further, beside translation and rotation, scaling may be used, particularly a similarity transformation. Thus, in embodiments, a non-rigid transformation may be used, being adapted to provide accurate results.

In some examples, it may also be possible that the mapping is non-rigid.

In another embodiment, the determining of the size of the nidus may comprise identifying an artery and/or a vein in the radiography image and determining a location of the nidus in between the identified artery and the identified vein. In particular, in some situations, contrast may be injected and there are phases where arteries are more visible than veins and vice-versa. Moreover, the location of the void may imply the location of the nidus. The size of nidus has more clinical relevance. The location of the lesion may be used to determine proximity to eloquent areas.

In some examples, the radiography image may be provided as a digital subtraction angiography, DSA, image.

In some examples, a raw radiography image and a segmented image (e.g., a radiography image onto which segments of the atlas are mapped) may be retrieved from a same or different remote entities (e.g., through a secure channel). In particular, a secure channel may include whitelisted IP address enabling the transfer of data.

By determining the location of the nidus, a location of a potential lesion may be further specified based on which a subsequent grading of the lesion may be performed.

In another embodiment, the lesion may be an arteriovenous malformation, AVM.

An AVM may be referred to as a congenital vascular abnormality characterized by a tangled network of blood vessels where arteries connect directly to veins (e.g., arising from a formation of a nidus) without the usual intervening capillary bed. This abnormal connection may create a high-pressure, high-flow system that bypasses normal tissue oxygenation. The absence of capillaries in the malformation can lead to weakened blood vessel walls, increasing the risk of rupture and subsequent hemorrhage.

By means of the computer-implemented method set out herein, an improved and automated characterization of an AVM may be facilitated.

In another embodiment, the computer-implemented method may further comprise associating the lesion, based on the determined size of the nidus, with a grade of a grading system, preferably of a Spetzler-Martin grading system, wherein the grading system indicates a suitability of the lesion and the patient for a treatment of the lesion by surgery.

In some examples, the grading system may comprise five grades, wherein grade I may be associated with a minimal surgical risk and wherein grade V may be associated with a very high surgical risk, often considered as inoperable.

In some examples, the Spetzler-Martin grading system may be referred to as a classification system for quantifying a surgical risk of AVMs.

This may enable an accurate and automized determination, whether a lesion may be treated by surgery or not.

In another embodiment, the computer-implemented method may further comprise suggesting a treatment for the lesion based at least in part on the determined grading of the lesion and preferably based at least in part on one or more of an age of a patient, country specific guidelines for treating the lesion and/or a parameter that is used to determine the grading.

The country specific guidelines may refer to a recommended treatment procedure for treating the lesion in case certain preconditions (e.g., the determined grading of the lesion) are present.

The parameter that is used to determine the grading may be one or more of the determined distance of the void to the region of interest, based on the size of the nidus and/or based on the determined presence of the deep venous drainage.

In another embodiment, the suggesting of the treatment may comprise querying a database for patients with a similar grading and their respective administered treatment.

A grading may be referred to as similar if the grading differs from the grading, determined by the computer-implemented method as set out herein, by +/- 1 grading step, e.g., on a grading scale which spans across grading steps from 0 to 5.

An administered treatment may be a treatment that has historically been assigned to a patient with a respective grading step.

This may support an improved decision making when suggesting a treatment for a lesion of a blood vessel of a patient. This may be facilitated as historic administered treatments and their potential outcomes may be taken into consideration when suggesting the treatment for the patient at the present time.

In another embodiment, the computer-implemented method may further comprise storing the suggested treatment to a database.

In some examples, the suggested treatment may be stored together with the determined grade.

The database may be located at a remote entity, i.e., an entity that is spatially different from the entity that is configured to execute the computer-implemented method as set out herein.

This may efficiently support an increase of the dataset stored in the database and may thus support and improve a subsequent and tailored suggesting of a treatment for an identified lesion.

According to a second aspect, a computer program product is suggested. The computer program product may comprise instructions which, when the program is executed by a computer, cause the computer to carry out the computer-implemented method as set out above.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a third aspect, a computer-implemented apparatus for characterizing a lesion in a blood vessel depicted in a radiography image is suggested. The computer-implemented apparatus may comprise an identifying unit configured for identifying a void, in the radiography image, that is representative for a presence of the lesion, a determining unit configured for determining a distance of the identified void to a region of interest in the radiography image, a determining unit configured for determining a size of a nidus located in between the identified artery and the vein, a determining unit configured for determining a presence of a deep venous drainage in the radiography image and a determining unit configured for determining a grading of the lesion based on the determined distance of the void to the region of interest, based on the size of the nidus and/or based on the determined presence of the deep venous drainage.

According to an aspect, the computer-implemented apparatus may further comprise an execution unit for executing the computer-implemented method as set out above.

Each of the units mentioned herein may be provided as or may comprise a respective processor (e.g., as a central processing unit (CPU)) and/or a field programmable gate array (FPGA)) and/or a graphics processing unit (GPU) and/or a tensor processing unit (TPU). In some examples, the respective units may comprise a memory.

According to a fourth aspect, a system for characterizing a lesion in a blood vessel depicted in a radiography image is suggested. The system may comprise the computer-implemented apparatus as set out above and the computer-program product as set out above.

Even though some embodiments are described in isolation from each other, it is emphasized that the embodiments may nevertheless be combined with each other.

The embodiments and features described with reference to the apparatus of the present invention apply, *mutatis mutandis,* to the method of the present invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Fig. 1: depicts an exemplary method for identifying a void;
- Fig. 2: depicts an exemplary method for determining a distance;
- Figs. 3A and 3B: depict an exemplary method for differentiating feed arteries from draining veins;
- Fig. 4: depicts an exemplary method for semi-automatically segmenting arteries;
- Fig. 5: depicts an exemplary method for determining a size of a nidus;
- Figs. 6A and 6B: depict an exemplary method for determining a presence of a deep venous drainage;
- Fig. 7: depicts an exemplary method for determining a grading of the lesion;
- Fig. 8: depicts an exemplary method for suggesting a treatment of a lesion;
- Fig. 9: depicts a workflow of a computer-implemented method for characterizing a lesion in a blood vessel;
- Fig. 10: depicts an exemplary computer-implemented apparatus for characterizing a lesion in a blood vessel; and
- Fig. 11: depicts an exemplary system for characterizing a lesion in a blood vessel.

In the Figures, like reference numerals designate like or functionally equivalent elements, unless otherwise indicated.

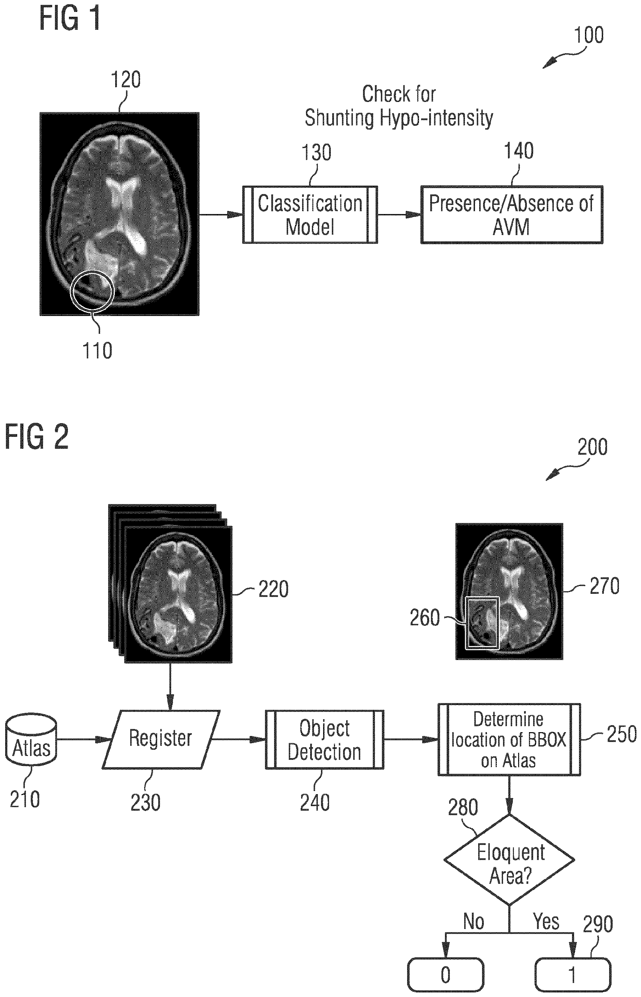

Fig. 1 depicts an exemplary method 100 for identifying a void 110 in a radiography image 120, e.g., an MRI.

The radiography image 120 may be provided to a trained artificial intelligence model 130, which may preferably be implemented as a classification model. The trained artificial intelligence model 130 may have been trained to identify a void in the radiography image 120.

An output of the trained artificial intelligence model 130 may indicate a presence/absence of AVM 140 in the radiography image 120. In this regard, it may be noted that a void in T2 MRI depicts a low resistance pathway for the blood to flow. In particular, not all voids mean AVMs. But, the first AI model (ResNet) based one may be trained to classify voids arising from AVMs as positive class. The model could be modified to output three classes namely, "void due to AVM", "void due to other pathological condition", and "no void".

In some examples, the presence of the AVM may be indicated by the trained artificial intelligence model 130 by outputting a logical 1 (or any other suitable indicator). In some examples, the absence of the AVM may be indicated by the trained artificial intelligence model 130 by outputting a logical 0 (or any other suitable indicator).

In case the lesion is an AVM, AVMs may oftentimes lack capillary components that may lead to arterialized veins in a high-flow, low-resistance shunt system. A fast blood flow may generate flow voids and may thus be seen on T2 weighted magnetic resonance images which are generally hypo-intense in nature.

In MRI, hypo-intense may refer to areas or tissues that appear darker compared to surrounding tissues on an image. This lower signal intensity is relative to the adjacent structures and can vary depending on the specific MRI sequence used. In some examples, on T2-weighted images, hypo-intense regions can suggest certain tumors, dense tissue such as calcified lesions, or iron deposits.

Fig. 2 depicts an exemplary method 200 for determining a distance of the identified void (e.g., according to the method 100) to a region of interest in the radiography image.

In some examples, an atlas 210 may be provided. An atlas may be referred to as a standardized reference framework that maps, names and delineates various anatomical and functional regions within, e.g., the brain of the patient.

Subsequently, a plurality of radiography images 220 (e.g., MRI) may be provided and registered 230. In some cases, only a single radiography image 220 may be provided and registered 230. In particular, registering may include transforming two images into one coordinate system.

Subsequently, an object detection 240 may be executed. The object detection 240 may be configured to determine a plurality of objects such as anatomical regions in the provided plurality of radiography images 220. The determining may, e.g., comprise identifying the void in the provided plurality of radiography images 220. In some examples, the determining of the plurality of objects may further comprise a determining of respective locations of the determined plurality of anatomical regions.

In an example, as part of the object detection 240, a box 260 may be drawn about a determined object in a radiography image 270 to highlight the respective determined object.

Subsequently, the objects determined as part of the object detection 240 (as well as their location) may be compared 250 to the atlas 210. The comparing may comprise a mapping of anatomical regions represented in the atlas 210 onto the objects determined as part of the object detection 240. In other words, the objects determined as part of the objection detection 240 may be associated with respective anatomical regions set out in the atlas 210. This may effectively lead to an annotation and labeling, respectively, of the objects determined as part of the object detection 240 and of the object, highlighted by box 260.

Based on the mapping, a determining 280 is performed, whether an object determined as part of the object detection 240 lies in area region of interest such as, e.g., an eloquent area.

The result of the determining 280, whether an object determined as part of the object detection 240 lies in a region of interest or not is output 290 (e.g., on a screen and/or as a flag and/or as a logical number which may subsequently be further processed).

In case the result of the determining 280 is output 290 as a logical number, a logical 1 may be output if it is determined 280 that the object lies in a region of interest. Alternatively, a logical 0 may be output if it is determined 280 that the object does not lie in a region of interest.

In case it is determined that the object lies in a region of interest (or is distanced from a region of interest by a metric separation that lies below a predetermined threshold), a treatment of the lesion may be suggested that is non-invasive to keep a potential risk for the patient as low as possible.

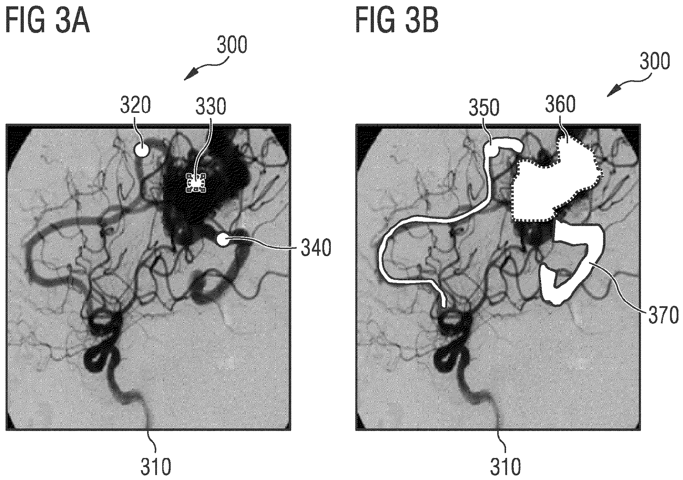

Figs. 3A and 3B depict an exemplary method 300 for differentiating feed arteries from draining veins in a radiography image 310.

More specifically, Fig. 3A depicts an exemplary radiography image 310 superimposed by a user input that indicates reference points 320, 330 and 340 which reference points 320, 330 and 340, in turn, associate structures of the radiography image 310 to arteries/veins and a nidus depicted in the radiography image 310.

In some examples, a single reference point is provided for each artery, vein and nidus depicted in the radiography image 310. In some examples, more than one reference point may be provided for each artery, vein and nidus depicted in the radiography image 310.

Fig. 3B depicts the exemplary radiography image 310 of Fig. 3A, wherein the artery, vein and the nidus depicted thereon has been determined (i.e., identified) and highlighted.

Based on the user input (i.e., based on the reference points 320, 330 and 340), the arteries 350, veins 370 and the nidus 360 may be determined and highlighted, e.g., by a trained artificial intelligence model that has been trained to determine arteries, veins and/or a nidus in a radiography image based on initially provided reference points.

In some examples, the respective highlighting may comprise a contouring (e.g., drawing a demarcation line) of the arteries, veins and the nidus. In some examples, the highlighting may comprise a filling of the respective arteries, veins and the nidus with color.

This may effectively provide a semi-automatic segmentation procedure for determining arteries, veins and/or a nidus based on an initially provided user input such as providing the reference points 320, 330 and 340.

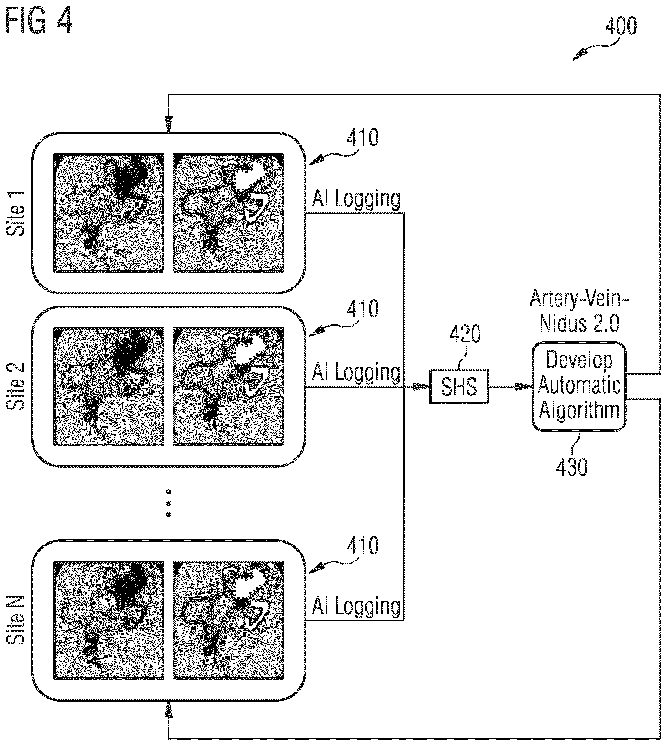

Fig. 4 depicts an exemplary method 400 for semi-automatically segmenting arteries, veins and a nidus in a radiography image, e.g., the radiography image 310.

As outlined with respect to Fig. 3, above, the segmentation may be based on reference points provided, e.g., by a user (e.g., medical practitioner), that indicate a location in a radiography image which depicts an artery, a vein and/or a nidus.

The exemplary procedure 400 may be based on retrieving a plurality of historically associated pairs of reference points and arteries, veins and/or a nidus 410, e.g., from a remote entity. The plurality of historically associated pairs of reference points and arteries, veins and/or a nidus 410 may comprise pairs of images wherein a respective pair comprises a radiography image with reference points and an image with a respectively segmented artery, vein and/or a nidus based on the reference points.

In some examples, the retrieving may comprise an logging procedure to collect interaction, such as, e.g., click points.

The plurality of historically associated pairs of reference points and arteries, veins and/or a nidus 410 may be retrieved through a secure channel 420.

The pairs of reference points and arteries, veins and/or a nidus 410 may be retrieved by an algorithm 430. In some examples, the algorithm 430 may be provided as a trained artificial intelligence model which may be trained (based at least in part on the retrieved pairs of reference points and arteries, veins and/or a nidus 410) to determine an anatomical structure of arteries, veins and/or a nidus in a radiography image based on initially provided reference points.

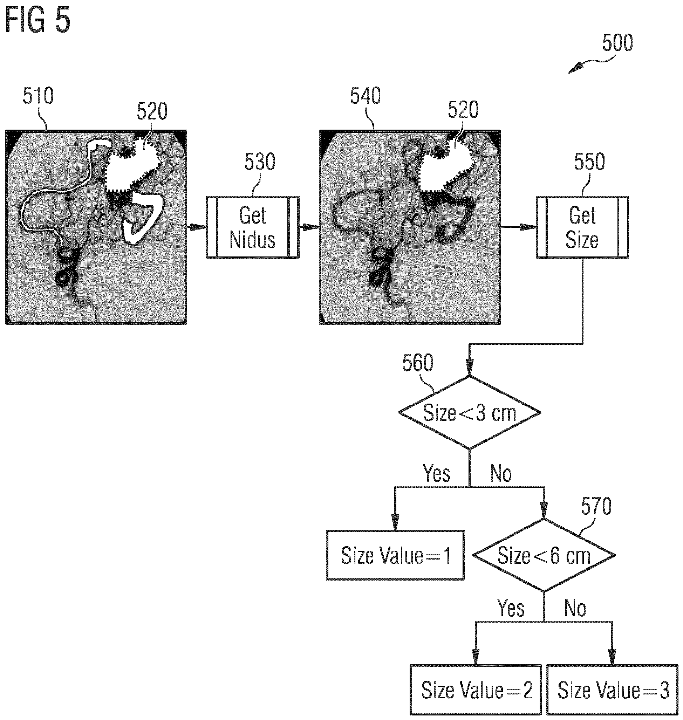

Fig. 5 shows an exemplary method 500 for determining a size of a nidus in a radiography image 510.

The radiography image 510 may be provided. A nidus 520 may have been determined and highlighted in the radiography image 510, e.g., according to at least one of the methods 300 and/or 400 as outlined above.

Subsequently, a nidus segmentation 530 may be performed. The nidus segmentation 530 may delete other anatomical regions in the radiography image 510 which were not determined as a nidus 520 in at least one of the methods 300 and/or 400.

That is, a reduced radiography image 540 may be provided that only depicts the nidus 520 as a highlighted anatomical region.

Subsequently, a size 550 may be derived from the radiography image 540.

Afterwards, it may be determined 560, whether the size of the nidus 520 exceeds a first predefined threshold (e.g., whether the size of the nidus 520 exceeds 3 cm or not). The first predefined threshold may be defined by an underlying grading system, such as, e.g., the Spetzler-Martin grading system (but not limited thereto).

If it is determined that the nidus is smaller than the first predefined threshold, a respective output may be provided (e.g., a 1 or any other suitable number).

If it is determined that the size of the nidus 520 exceeds the first predefined threshold, it may be determined 570 whether the size of the nidus 520 exceeds a second predefined threshold (e.g., whether the size of the nidus 520 exceeds 6 cm or not).

If it is determined that the size of the nidus 520 is smaller than the second predefined threshold, a respective output may be provided (e.g., a 2 or any other suitable number).

If it is determined that the size of the nidus 520 is larger than the second predefined threshold, a respective output may be provided (e.g., a 3 or any other suitable number).

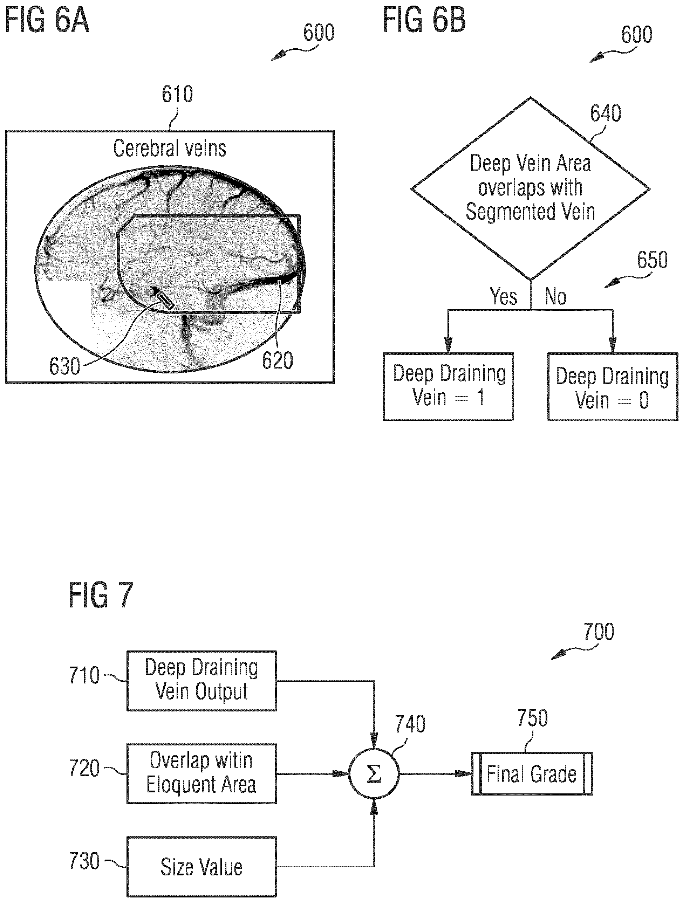

Figs. 6A and 6B show an exemplary method 600 for determining a presence of a deep venous drainage in a radiography image (e.g., the radiology image 310, 510).

Fig. 6A depicts a radiography image 610 (e.g., a DSA image).

The method 600 may be initiated by providing the radiography image 610, e.g., depicting cerebral veins.

The radiography image 610 may be provided with a highlighted region, indicating a potential deep vein area 620 that indicates a potential region in the radiography image 610 in which the probability that deep veins may be found is higher than in a surrounding region. The potential deep vein area 620 may be provided by a medical practitioner (e.g., a radiologist). The potential deep vein area 620 may be provided manually (e.g., by a medical practitioner).

In some examples, veins (or a single vein) depicted in the radiography image 610 may have been segmented beforehand. In some examples, the veins may have been segmented according to any of the methods 300 and/or 400 as set out with reference to Figs. 3 and 4, above. In some examples, veins may have been segmented according to another segmentation procedure which is not expressly disclosed herein.

The segmented veins 630 may be overlaid onto the radiography image 610.

In case an overlay arises between the segmented veins 630 and the potential deep vein area 620, the segmented veins 630 are referred to as deep veins.

Deep lying veins may, e.g., be one or more of a sigmoid sinus, a straight sinus and/or a lateral sinus.

Veins such as superior sagittal sinus, superior cerebral veins, sphenoparietal sinus may be referred to as superior veins.

Fig. 6B shows a flowchart illustrating the determining of the presence of the deep venous drainage in the radiography image 610.

At first, it may be determined 640, whether the potential deep vein area 620 overlaps with the segmented veins 630.

Based thereon, an output 650 may be provided.

In some examples, if it is determined that the deep vein area overlaps with the segmented veins, a logical 1 may be output. However, it may also be possible that any other number may be output that indicates an overlap.

In some examples, if it is determined that the deep vein area does not overlap with the segmented veins, a logical 0 may be output. However, it may also be possible that any other number may be output that indicates an absence of an overlap.

Fig. 7 depicts an exemplary method 700 for determining a grading of the lesion.

The method 700 may comprise providing information 710, associated with the presence of a deep venous drainage, to a computation unit 740.

The method 700 may comprise providing information 720, associated with the determining of a distance of the identified void to the region of interest in the radiography image, to the computation unit 740.

The method 700 may comprise providing information 730, associated with the determining of the size of the nidus in the radiography image, to the computation unit 740.

The computation unit 740 may be configured to calculate a grading 750 of the lesion based on one or more the information 710 - 730 provided to the computation unit 740.

In some examples, the provided information 710 - 730 may each refer to a number that had been assigned as a result of executing a respective method 200, 500 and/or 600 as set out above. In some examples, the computation unit 740 may be configured to calculate a sum of the numbers provided to the computation unit 740. In some examples, the numbers may be weighted relative to each other.

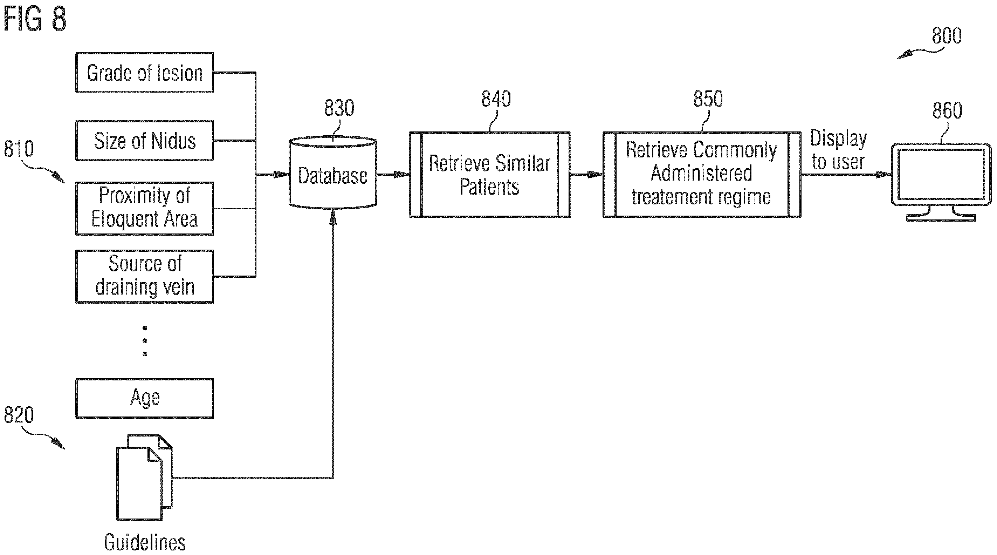

Fig. 8 depicts an exemplary method 800 for suggesting a treatment of a lesion.

The method 800 may comprise providing information associated with the lesion 810 to a database 830. In some examples, the information associated with the lesion 810 may comprise a grade of a lesion, a size of a nidus, a distance between a void and a region of interest, a segmented vein, an age of a patient, etc.

The method 800 may comprise providing (national) guidelines 820 to the database 830.

Based thereon, information associated with similar patients 840 (relative to the patient that is associated with the information provided to the database 830) may be retrieved from the database 830 and may be provided to a medical practitioner.

Based on the information associated with similar patients 840, retrieved from the database 830, a number K, with K ≥ 1, of top treatment protocols may be derived 850 that were administered to the similar patients.

In some examples, said treatment protocols may be provided in a ranked list (e.g., ranked in descending or ascending order with respect to a historically achieved success rate of a respective treatment protocol). In some examples, the ranked list may be ranked according to an expected success rate of a treatment if the treatment is carried out according to a respective treatment protocol. The expected success rate may be calculated by a patient model (e.g., based on a respectively trained artificial intelligence model that may have been trained to determine an excepted success rate of a treatment which may be suggested to treat a lesion).

The resulting suggested treatment (e.g., provided as a treatment protocol) may be displayed to a user 860.

In some examples, it may be possible that the suggested treatment for a patient may be stored to the database 830 (incl. relevant information that was used for determining the suggested treatment). This may improve a quality of information that is stored in the database over the course of time and may make the database 830 growing over time. Due to a growing database, based on which a treatment may be suggested for a patient, the suggesting of a treatment may more closely be tailored to the patient while still being compliant to respective (national) guidelines.

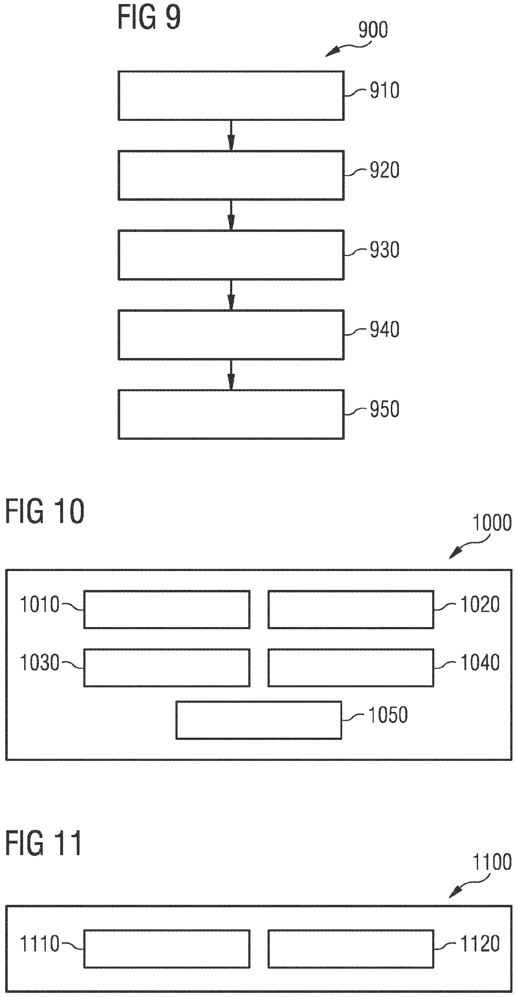

Fig. 9 depicts a workflow of a computer-implemented method 900 for characterizing a lesion in a blood vessel of a brain depicted in a radiography image.

In step 910, a void is identified, in the radiography image, wherein the void is representative for a presence of the lesion.

In step 920, a distance is determined of the identified void to a region of interest in the radiography image.

In step 930, a size of a nidus in the radiography image is determined.

In step 940, a presence of a deep venous drainage in the radiography image is determined.

In step 950, a grading of the lesion is determined based on the determined distance of the void to the region of interest, based on the size of the nidus and/or based on the determined presence of the deep venous drainage.

Fig. 10 shows an exemplary computer-implemented apparatus 1000 for characterizing a lesion in a blood vessel depicted in a radiography image. The apparatus 1000 comprises an identifying unit 1010, a determining unit 1020 for determining a distance, a determining unit 1030 for determining a size, a determining unit 1040 for determining a presence and a determining unit 1050 for determining.

The identifying unit 1010 is configured for identifying a void in the radiography image, that is representative for a presence of the lesion.

The determining unit 1020 is configured for determining a distance of the identified void to a region of interest in the radiography image.

The determining unit 1030 is configured for determining a size of a nidus located in between the identified artery and the vein.

The determining unit 1040 is configured for determining a presence of a deep venous drainage in the radiography image.

The determining unit 1050 is configured for determining a grading of the lesion based on the determined distance of the void to the region of interest, based on the size of the nidus and/or based on the determined presence of the deep venous drainage.

Fig. 11 shows an exemplary system 1100 for characterizing a lesion in a blood vessel depicted in a radiography image.

The system 1100 may comprise the computer-implemented apparatus as disclosed herein, e.g., the computer-implemented apparatus 1000.

The system 1100 may comprise the computer-program product as disclosed herein.

Although the present invention has been described in accordance with preferred embodiments, it is obvious for the person skilled in the art that modifications are possible in all embodiments.

### REFERENCE NUMERALS

- 100: method
- 110: void
- 120: radiography image
- 130: trained artificial intelligence model
- 140: AVM
- 200: method
- 210: atlas
- 220: radiography image
- 230: registering step
- 240: object detection
- 250: comparison step
- 260: box
- 270: radiography image
- 280: determining step
- 290: output
- 300: method
- 310: radiography image
- 320: reference point
- 330: reference point
- 340: reference point
- 350: highlighted artery
- 360: highlighted nidus
- 370: highlighted vein
- 400: method
- 410: historically associated pairs
- 420: secure channel
- 430: algorithm
- 500: method
- 510: radiography image
- 520: nidus
- 530: nidus segmentation
- 540: radiography image
- 550: size
- 560: determination step
- 570: determination step
- 600: method
- 610: radiography image
- 620: potential deep vein area
- 630: segmented veins
- 640: determining step
- 650: output
- 700: method
- 710: information associated with a presence of a deep venous drainage
- 720: information associated with determining a distance
- 730: information associated with determining a size
- 740: computation unit
- 750: grading
- 800: method
- 810: lesion
- 820: guidelines
- 830: database
- 840: information associated with similar patients
- 850: derived treatment protocols
- 860: user
- 900: computer-implemented method
- 910: step
- 920: step
- 930: step
- 940: step
- 950: step
- 1000: computer-implemented apparatus
- 1010: identifying unit
- 1020: determining unit for determining a distance
- 1030: determining unit for determining a size of a nidus
- 1040: determining unit for determining a presence
- 1050: determining unit for determining a grading
- 1100: system
- 1110: computer-implemented apparatus
- 1120: computer-program product

## Claims

1. A computer-implemented method (900) for characterizing a lesion in a blood vessel of a brain depicted in a radiography image, comprising:
identifying (910) a void in the radiography image, wherein the void is representative for a presence of the lesion;
determining (920) a distance of the identified void to a region of interest in the radiography image;
determining (930) a size of a nidus in the radiography image;
determining (940) a presence of a deep venous drainage in the radiography image; and
determining (950) a grading of the lesion based on the determined distance of the void to the region of interest, based on the size of the nidus and/or based on the determined presence of the deep venous drainage.

2. The computer-implemented method of claim 1, wherein the identifying of the void comprises identifying the void based on a trained artificial intelligence model, preferably by a trained classification model, more preferably by a Residual Network, ResNet.

3. The computer-implemented method of any of the preceding claims, wherein the region of interest is an eloquent area of the brain.

4. The computer-implemented method of any of the preceding claims, wherein the determining of the distance comprises:
mapping an atlas of the brain to the radiography image;
providing the mapped radiography image to a trained artificial intelligence model which is trained for localizing a lesion in a radiography image; and
localizing the lesion in the radiography image, based on the respectively trained artificial intelligence model, wherein the localizing preferably comprises determining coordinates of the lesion.

5. The computer-implemented method of claim 4, wherein the mapping of the atlas comprises a rigid mapping.

6. The computer-implemented method of any of the preceding claims, wherein the determining of the size of the nidus comprises:
identifying an artery and/or a vein in the radiography image;
determining a location of the nidus (306) in between the identified artery (350) and the identified vein (370).

7. The computer-implemented method of any of the preceding claims, wherein the lesion is an arteriovenous malformation, AVM.

8. The computer-implemented method of any of the preceding claims, further comprising:
associating the lesion, based on the determined size of the nidus, with a grade of a grading system, preferably of a Spetzler-Martin grading system, wherein the grading system indicates a suitability of the lesion and the patient for a treatment of the lesion by surgery.

9. The computer-implemented method of any of the preceding claims, further comprising:
suggesting a treatment for the lesion based at least in part on the determined grading of the lesion and preferably based at least in part on one or more of an age of a patient, country specific guidelines for treating the lesion and/or a parameter that is used to determine the grading.

10. The computer-implemented method of claim 9, wherein the suggesting of the treatment comprises:
querying a database for patients with a similar grading and their respective administered treatment.

11. The computer-implemented method of any of the claims 9 or 10, further comprising:
storing the suggested treatment to a database.

12. A computer program product comprising instructions which, when the program is executed by a computer, cause the computer to carry out the computer-implemented method of one of the claims 1 to 11.

13. A computer-implemented apparatus (1000) for characterizing a lesion in a blood vessel depicted in a radiography image, comprising:
an identifying unit (1010) configured for identifying a void, in the radiography image, that is representative for a presence of the lesion;
a determining unit (1020) configured for determining a distance of the identified void to a region of interest in the radiography image;
a determining unit (1030) configured for determining a size of a nidus located in between the identified artery and the vein;
a determining unit (1040) configured for determining a presence of a deep venous drainage in the radiography image; and
a determining unit (1050) configured for determining a grading of the lesion based on the determined distance of the void to the region of interest, based on the size of the nidus and/or based on the determined presence of the deep venous drainage.

14. The computer-implemented apparatus of claim 13, further comprising:
an execution unit for executing the computer-implemented method according to one of the claims 1 to 12.

15. A system (1100) for characterizing a lesion in a blood vessel depicted in a radiography image, comprising:
the computer-implemented apparatus (1110) of any of the claims 13 or 14; and
the computer-program product (1120) of claim 12.
